# EUROPEAN PATENT SPECIFICATION

(11) **EP 1 722 733 B1**
(45) Date of publication and mention of the grant of the patent: **20.06.2007**
(21) Application number: 04729164.6
(22) Date of filing: 23.04.2004
(51) Int. Cl.: A61F 9/06

(54) **AN IMPROVED WELDING MASK**
VERBESSERTE SCHWEISSENMASKE
MASQUE DE SOUDAGE AMELIORE

(30) Priority: 12.01.2004 IT TO20040009
(43) Date of publication of application: 22.11.2006
(73) Proprietor: Elto S.P.A., 10094 Giaveno (Torino) (IT)
(72) Inventor: LOEWENTHAL, Enrico, I-10090 Rivoli (Torino) (IT)
(74) Representative: Deambrogi, Edgardo
(86) International application number: PCT/IB2004/001234
(87) International publication number: WO 2005/074849

(56) References cited:
- CH-A- 197 104
- DE-U- 29 602 946
- US-A- 4 039 254
- US-A- 4 039 803
- US-A- 4 237 557
- US-A- 4 241 286

## Description

The present invention relates to an eye protection mask for persons carrying out arc welding operations.

It is well known that the intensity of the light emitted by an electric arc during arc welding can cause damage to the eyesight if the arc and the working area directly illuminated by it are viewed with the naked eye. Masks have therefore long been provided which have a window glazed with a highly absorbent glass, known as non-actinic glass, enabling an operator to observe the area where he is performing an arc weld without any danger to his sight.

While the earliest of these masks had a fixed non-actinic glass and had to be removed from the user's field of view in order to observe the area being worked on when the arc was switched off, masks were later introduced which had a movable non-actinic glass, which the operator could move between an active position in the line of sight and an inactive position out of the line of sight by means of a lever trigger. The operator is thus able to observe the area on which he is working both when the arc is on and when it is off, simply by remembering each time to move the non-actinic glass into the appropriate position. Manipulation of these mechanical devices requires some force however, and it is easy for the operator's finger to slip on the lever while the arc is active, thereby causing a period without protection and the resulting damages. In addition, the mechanical device easily jams and malfunctions, as a result of the considerable transmission ratio required between the lever and the mechanism which moves the non-actinic glass.

Masks for arc welding were introduced recently in which the observation window is covered by a liquid crystal screen which is normally transparent but which becomes highly absorbent, much like non-actinic glass, on application of a particular signal. These masks have a photoelectric cell which, when affected by light from the arc, activates a circuit which signals the liquid crystal screen to darken. In this way, protection is activated entirely automatically simply by switching on the arc, and the speed of reaction of the device is enough to limit the time elapsed between activation of the arc and the darkening of the liquid crystal screen to an amount so brief to prevent damages to the operator's sight.

These masks do have some fundamental disadvantages however. A first disadvantage consists in the fact that the resting condition of the mask corresponds to the liquid crystal screen in its transparent state. This means that any circuit fault, or a flat power supply battery or the accidental presence of an object preventing the arc light from reaching the photoelectric cell, will lead to a lack of protection and thus to the resulting damages. Another disadvantage lies in the fact that the device must be kept in operating condition, and therefore using energy, for the entire period during which welding is carried out, with the result that the life of the power supply batteries is short, even if relatively expensive ones are used. Another serious disadvantage consists in the high cost of such masks, as a result of the relative complexity of the optoelectronic circuits and the components used.

The object of the present invention is therefore to provide a welding mask that overcomes the aforesaid disadvantages of masks known in the art. In particular, one object of the invention is to provide a welding mask in which no accidental occurrence, such as a fault or a flat battery, will under any circumstances lead to loss of protection. Another object of the invention is to provide the operator with a control of the condition of the mask which can be operated extremely simply and with no effort. Yet another object of the invention is to provide operation with a minimum consumption of electrical power, ensuring that the power supply batteries last longer, even if they are of an economy type. A final object of the invention is to enable the mask to be manufactured at relatively low cost.

These objects are achieved in an arc welding mask with an observation window covered by a liquid crystal screen, which can be switched to a substantially transparent condition or to a condition of high absorbency, characterised in that the liquid crystal screen is arranged to be in the condition of high absorbency when in its non-activated state and to switch to the substantially transparent condition as a result of being activated.

Preferably, the electrical circuit controlling the state of the liquid crystal screen is arranged to remain totally inactive during all those periods when the operator has not controlled it to obtain the transparent condition of the liquid crystal screen in order to observe the work area without light being absorbed.

As a result of these arrangements, any occurrence leading to inactivity of the electrical control circuit, such as a fault, a flat power supply battery or the operator's finger accidentally slipping away from the activation control, causes the liquid crystal screen to revert to its darkened condition, thus ensuring protection. In addition, since the electrical circuit is not in permanent operation, but is activated only under certain circumstances, energy consumption is low and the power supply batteries remain charged for a long time, so that an economy type of battery can be used. The operator can carry out the control by a simple push-button, thus in a very easy way. Finally, since no optoelectronic means are required to detect the light from the arc, the cost of the device is substantially reduced.

The darkened condition corresponding to the non-activated state of the liquid crystal screen may be easily reached in various ways, for example by superimposing at least one layer of liquid crystals and a permanent polarising layer with its polarisation axis orthogonal to the polarisation axis of the liquid crystals in their rest state, or by superimposing two or more layers of liquid crystals operable to react in the opposite way when activated or, yet again and preferably, by means of a screen with three layers, two outer layers that are either permanently linearly polarised, with crossed polarisation axes or circularly polarised with opposite senses of rotation, and an intermediate layer of liquid crystals which are optically inactive when not activated and optically polarised when activated.

In order for it to be really easily operated, the control button can be fitted into the handle for gripping the mask, which can also contain the power supply batteries and the electronic control circuit.

These and other characteristics, objects and advantages of the subject of the invention will become more apparent from the following description of an exemplary but non-limitative embodiment, with reference to the appended drawing, in which:
- Figure 1 is an elevation view of the mask facing towards the operator;
- Figure 2 is a vertical section taken on the line II-II of Figure 1;
- Figure 3 is a block diagram of the electrical circuit controlling the state of the liquid crystal screen;
- Figure 4 is a diagram of a signal that can be used to control the state of the liquid crystal screen.

The mask shown has a body 1 substantially in the shape of a shield, with a grip handle 2. The body 1 must be opaque to light and can be made of a suitable plastics material, metal or other suitable material. A viewing window 3 is formed in the body 1, being sufficiently large for an operator to observe the area he is working on while carrying out an arc welding operation. The viewing window 3 is fitted with a liquid crystal screen 4. This screen 4, according to the various possible embodiments, may be constituted by a single layer or by several layers of different or differently orientated materials. Its fundamental characteristic, according to the invention, is that when it is not activated it is in its darkened condition.

A cavity is formed in the handle 2 for housing an electrical circuit 5 including suitable power supply batteries. This circuit is connected to the liquid crystal screen 4 by connectors 6. According to a preferred characteristic of the invention, the electrical circuit 5 is operable to remain totally inactive under normal circumstances and to be activated by controlling a push-button 7 arranged in the handle 2.

In its rest state, therefore, the mask 1 has its viewing window 3 darkened by the liquid crystal screen 4 which, when not activated, is highly light absorbent. The mask can therefore be used to protect the eyes while an arc welding operation is being carried out and, since this occurs in the assembly's rest state, no kind of accident can compromise safety conditions.

If the operator wants to view the area he is working on while the arc is switched off, he may simply press the button 7 and the electrical circuit 5 sends a signal to the liquid crystal screen 4 which activates it, causing it to become substantially transparent. The operator is then able to observe the work area in the normal ambient light. It should be noted that any accidental occurrence which is to prevent correct operation of the assembly, prevents this action and makes the operator move the mask in order to examine the area he is working on, but the protective effect of the mask will in no way be compromised.

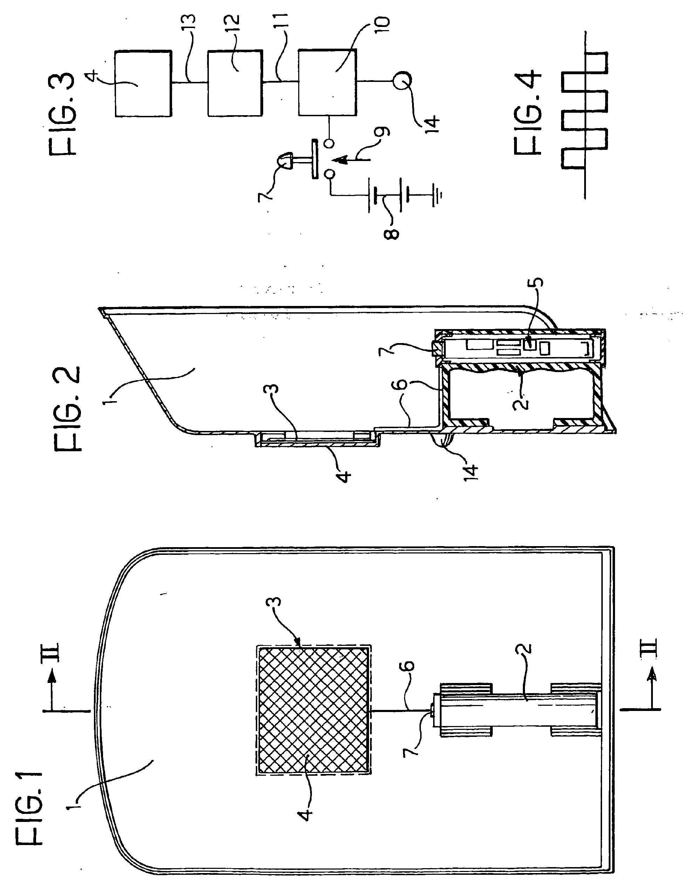

The electrical circuit 5 can be shown in a block diagram such as that given in Figure 3. It includes a power supply battery 8, the activation button 7 which is normally held in its open condition by a resilient force indicated by the arrow 9, a voltage-boost stage 10, which makes it possible to use batteries 8 providing a low voltage which would otherwise not be sufficient to power the control circuit, and an oscillator stage 12 which, when activated, produces a signal operable to activate the liquid crystal screen 4. For example, two 1.5 volt AA batteries could be used, providing d.c. voltage of 10 volts at the point 11 for powering the oscillator 12, while at its output 13 this oscillator can emit a square-wave signal such as that illustrated in Figure 4, which might have a voltage of ± 5 volts and a frequency of 70 hertz. It is clear that these values are given purely by way of example of implementation and the technician designing the system could choose different values.

As mentioned earlier, there are various ways of obtaining a normally darkened liquid crystal screen which becomes substantially transparent when activated. One method which seems particularly well suited to this application consists in superimposing three layers, the two outermost of which are permanently polarised with orthogonal polarisation planes or opposite senses of rotation, and thus dark when viewed together, and a third, intermediate, layer between the first two, consisting of liquid crystals which are optically inactive when not activated and polarised when electrically activated. The polarisation of this third layer when it is activated shifts the plane or sense of polarisation of light coming through one of the outer layers so that the light is no longer intercepted by the other outer layer.

The mask as described thus far has no costly optoelectronic component operable to react to the light emitted by the electric arc and is therefore remarkably economical. However, if one accepts a slight increase in cost the mask of the invention can be further improved by providing a photoelectric cell 14 which can receive light from the arc and is arranged so as to inhibit operation of the electrical circuit 5. In this way, the photoelectric cell 14 protects against an erroneous move by the operator, who could inadvertently press the button 7 while the arc is on. It should be noted that the photoelectric cell would carry out the opposite function to that of a prior art automatic mask.

It is clear that the invention is not restricted to the embodiments described and illustrated by way of examples. Several possible modifications have been mentioned in this description, while others could be envisaged by an expert in this field.

## Claims

1. A mask for protecting the eyes of an operator carrying out arc welding, including a body (1) of an opaque material, shaped substantially like a shield, with a viewing window (3) fitted with a liquid crystal screen (4) which can be switched to a substantially transparent condition or to a condition of high absorbency, the liquid crystal screen (4) being arranged to be in a condition of high absorbency when in its non activated state, and to switch to the substantially transparent condition as a result of being activated by an electrical circuit (5), **characterised in that** it includes a grip handle (2) having a push button (7) for activating said electrical circuit (5).

2. A welding mask according to Claim 1, **characterised in that** the electrical circuit (5) controlling the state of the liquid crystal screen (4) is arranged to remain totally inactive throughout those periods when the operator does not control it to switch the liquid crystal screen (4) to its transparent condition, in order to observe the area being worked on without light being absorbed.

3. A welding mask according to Claim 1, **characterised in that** the grip handle (2) has a cavity for housing the electrical circuit (5) for controlling the state of the liquid crystal screen (4) as well as the power supply battery (8) .

4. A welding mask according to Claim 1, **characterized in that** the said liquid crystal screen (4) which is normally darkened and becomes substantially transparent when activated includes three layers, the outer two of which are permanently polarised, either with orthogonal polarisation planes or opposite senses of rotation, and therefore dark as an assembly, with a third layer, interposed between the first two, consisting of liquid crystals which are optically inactive when not activated and polarised when activated electrically.

5. A welding mask according to Claim 1, **characterised in that** the electrical circuit (5) controlling the state of the liquid crystal screen (4) includes a relatively low voltage power supply battery (8), a push button (7) which is normally open, a voltage-boosting stage (10), and an oscillator stage (12) operable to produce a signal for controlling the state of the liquid crystal screen (4).

6. A welding mask according to Claim 1, **characterised in that** it also includes a photoelectric cell (14) arranged so that light from an electric arc impinges thereon and is operable, when thus activated, to inhibit operation of the electrical circuit (5).

## Patentansprüche

1. Maske zum Schutz der Augen eines Arbeiters, der eine Lichtbogenschweißung durchführt, wobei die Maske einen Körper (1) aus einem lichtundurchlässigen Material aufweist, der im Wesentlichen schildartig ausgebildet ist und ein Fenster (3) besitzt, das mit einem Flüssigkeitskristall-Schirm (4) versehen ist, der in einen im Wesentlichen durchsichtigen Zustand oder in einen Zustand geschaltet werden kann, in dem er ein hohes Absorptionsvermögen besitzt, wobei der Flüssigkeitskristall-Schirm (4) so aufgebaut ist, dass er sich in einem Zustand mit einem hohen Absorptionsvermögen befindet, wenn er sich im deaktivierten Zustand befindet, und in den im Wesentlichen durchsichtigen Zustand umschaltet, wenn er von einem elektrischen Schaltkreis (5) aktiviert wird, **dadurch gekennzeichnet, dass** die Maske einen Handgriff (2) aufweist, der mit einer Drucktaste (7) versehen ist, um den elektrischen Schaltkreis (5) zu aktivieren.

2. Schweißmaske gemäß Anspruch 1, **dadurch gekennzeichnet, dass** der elektrische Schaltkreis (5), der den Zustand des Flüssigkeitskristall-Schirms (4) steuert, so aufgebaut ist, dass er über jene Zeitintervalle vollständig inaktiv bleibt, wenn der Arbeiter ihn nicht ansteuert, um den Flüssigkristall-Schirm (4) in dessen durchsichtigen Zustand zu schalten, um den Bereich, in dem gearbeitet wird zu beobachten, ohne dass Licht absorbiert wird.

3. Schweißmaske gemäß Anspruch 1, **dadurch gekennzeichnet, dass** der Handgriff (2) einen Hohlraum besitzt, um den elektrischen Schaltkreis (5) für die Steuerung des Zustands des Flüssigkristall-Schirms (4) sowie die Batterie für die Stromversorgung (8) aufzunehmen.

4. Schweißmaske gemäß Anspruch 1, **dadurch gekennzeichnet, dass** der Flüssigkristall-Schirm (4), der normalerweise abgedunkelt ist und dann im Wesentlichen durchsichtig wird, wenn er aktiviert wird, drei Schichten, von denen die beiden äußeren dauernd polarisiert sind, entweder in orthogonalen Polarisationsebenen oder in entgegengesetzten Drehrichtungen, wodurch sie als Aufbau dunkel sind, sowie eine dritte Schicht aufweist, die zwischen den beiden ersten Schichten liegt und aus Flüssigkristallen besteht, die optisch inaktiv sind, wenn sie nicht aktiviert werden, und polarisiert sind, wenn sie elektrisch aktiviert werden.

5. Schweißmaske gemäß Anspruch 1, **dadurch gekennzeichnet, dass** der elektrische Schaltkreis (5), der den Zustand des Flüssigkristall-Schirms (4) steuert, ein Batterie für die Stromversorgung (8) mit relativ niedriger Spannung, eine Drucktaste (7), die normalerweise offen ist, eine Spannungserhöhungs-Stufe (10) sowie eine Oszillatorstufe (12) aufweist, die in Betrieb gesetzt werden können, um ein Signal für die Steuerung des Zustands des Flüssigkristall-Schirms (4) zu erzeugen.

6. Schweißmaske gemäß Anspruch 1, **dadurch gekennzeichnet, dass** die Maske weiters eine fotoelektrische Zelle (14) aufweist, die so angeordnet ist, dass Licht von einem elektrischen Lichtbogen auf sie auftrifft, und die dann, wenn sie auf diese Weise aktiviert ist, in Betrieb gesetzt werden kann, um zu verhindern, dass der elektrische Schaltkreis (5) in Betrieb gesetzt wird.

## Revendications

1. Masque pour la protection des yeux d'un opérateur réalisant du soudage à l'arc, comprenant un corps (1) réalisé dans un matériau opaque, ayant sensiblement la forme d'un écran, avec une fenêtre de visualisation (3) équipée d'un écran à cristaux liquides (4) qui peut être commuté d'une condition sensiblement transparente à une condition d'absorbance élevée, l'écran à cristaux liquides (4) étant conçu pour être dans une condition d'absorbance élevée lorsqu'il se trouve à l'état non activé, et pour être commuté dans une condition sensiblement transparente à la suite de son activation par un circuit électrique (5), **caractérisé en ce qu'**il comprend une poignée (2) présentant un bouton-poussoir (7) destiné à activer ledit circuit électrique (5).

2. Masque de soudage selon la revendication 1, **caractérisé en ce que** le circuit électrique (5) commandant l'état de l'écran à cristaux liquides (4) est conçu pour rester totalement inactif pendant toutes les périodes où l'opérateur ne le commande pas pour commuter l'écran à cristaux liquides (4) dans sa condition transparente, afin d'observer la surface sur laquelle il travaille sans que la lumière soit absorbée.

3. Masque de soudage selon la revendication 1, **caractérisé en ce que** la poignée (2) présente une cavité pour loger le circuit électrique (5) destiné à commander l'état de l'écran à cristaux liquides (4) ainsi que le bloc d'alimentation électrique (8).

4. Masque de soudage selon la revendication 1, **caractérisé en ce que** ledit écran à cristaux liquides (4), qui est normalement noirci et devient sensiblement transparent lorsqu'il est activé, comprend trois couches, dont les deux plus externes sont polarisées de manière permanente, soit avec des plans de polarisation orthogonale soit des sens de rotation opposés, et forment donc un ensemble foncé, avec une troisième couche, interposée entre les deux premières, composée de cristaux liquides qui ne sont pas opto-actifs lorsqu'elle n'est pas activée et qui sont polarisés lorsqu'elle est activée électriquement.

5. Masque de soudage selon la revendication 1, **caractérisé en ce que** le circuit électrique (5) commandant l'état de l'écran à cristaux liquides (4) comprend un bloc d'alimentation à relativement basse tension (8), un bouton-poussoir (7) qui est normalement ouvert, un étage survolteur (10), et un étage oscillateur (12) actionnable pour générer un signal destiné à commander l'état de l'écran à cristaux liquides (4).

6. Masque de soudage selon la revendication 1, **caractérisé en ce qu'**il comprend également une cellule photoélectrique (14) disposée de telle sorte que la lumière provenant d'un arc électrique se heurte à celle-ci et est actionnable, lorsqu'elle est ainsi activée, pour empêcher l'actionnement du circuit électrique (5).
